# EUROPEAN PATENT APPLICATION

(11) **EP 2 687 152 A1**
(43) Date of publication of application: **22.01.2014**
(21) Application number: 13175346.9
(22) Date of filing: 05.07.2013
(51) Int. Cl.: A61B 5/00, A61B 5/022, G06F 19/00

(54) **Blood pressure measurement apparatus, gateway, system including the same, and method thereof**

(30) Priority: 20.07.2012 KR 20120079429
(71) Applicant: Samsung Electronics Co., Ltd, Gyeonggi-do 443-742 (KR)
(72) Inventor: Kim, Dong Woo, Gyeonggi-do (KR); Kim, Ji Eun, Gyeonggi-do (KR); Park, Jeong Je, Gyeonggi-do (KR); Lee, Kwang Hyeon, Seoul (KR)
(74) Representative: Hylarides, Paul Jacques

(57) **Abstract**

A blood pressure measurement apparatus for determining a level of rareness of raw data and transmitting the raw data to a gateway or a server according to the determined level of the raw data includes a blood pressure estimator configured to acquire raw data for estimating blood pressure, and to estimate blood pressure from the raw data according to a blood pressure estimation algorithm; and a controller configured to determine a level of rareness of the raw data, and to determine whether to transmit the raw data according to the determined level of rareness of the raw data.

## Description

### BACKGROUND

### 1. Field

One or more embodiments relate to a system and method for improving and developing a blood pressure estimation algorithm that is used for blood pressure measurement in a blood pressure measurement apparatus.

### 2. Description of the Related Art

In general, algorithms for blood pressure estimation of a blood pressure measurement apparatus have been developed based on database of raw data acquired upon development of the blood pressure measurement apparatus.

Accordingly, the performances of the algorithms depend on the amount and variety of raw data included in the database.

However, after an algorithm has been developed and installed in a product, raw data collected during measurement of blood pressure is used only as the results of measurement by the algorithm, that is, as base information for estimating blood pressure.

In order to develop an algorithm with improved performance, the variety, rareness, and representativeness of raw data configuring database are important factors. In order to obtain additional raw data, information should be acquired through a clinical trial, etc., which requires a significantly long time and high cost.

### SUMMARY

Therefore, the foregoing described problems may be overcome and/or other aspects may be achieved by one or more embodiments of a blood pressure measurement apparatus determining a level of rareness of raw data and transmitting the raw data to a gateway or a server according to the determined level of rareness of the raw data.

The foregoing described problems may be overcome and/or other aspects may be achieved by one or more embodiments of a gateway that may determine a level of rareness of raw data and may transmit the raw data to a server according to the determined level of rareness of the raw data.

The foregoing described problems may be overcome and/or other aspects may be achieved by one or more embodiments of a system that may include a server to determine a level of rareness of received raw data, allocate a weight to the raw data according to the determined level of rareness of the raw data, and determine whether to give a reward for data provision according to the weight.

Additional aspects and/or advantages of one or more embodiments will be set forth in part in the description which follows and, in part, will be apparent from the description, or may be learned by practice of one or more embodiments of disclosure. One or more embodiments are inclusive of such additional aspects.

In accordance with one or more embodiments, a blood pressure measurement apparatus may include: a blood pressure estimator configured to acquire raw data for estimating blood pressure, and to estimate blood pressure from the raw data according to a blood pressure estimation algorithm; and a controller configured to determine a level of rareness of the raw data, and to determine whether to transmit the raw data according to the determined level of rareness of the raw data.

The controller may determine the level of rareness of the raw data based on determination criteria including an age of a user using the blood pressure measurement apparatus, the user's medical history, a blood pressure measuring environment, and an aspect of the raw data.

The blood pressure measurement apparatus may further include a display unit configured to display the estimated blood pressure, and to receive a command from the controller to display a message for getting confirmation on whether to transmit the raw data.

The blood pressure measurement apparatus may further include a data transmitter configured to transmit the raw data if a command for approving transmission of the raw data is received.

The data transmitter may transmit the raw data to a server or a gateway.

In accordance with one or more embodiments, a gateway may include: a communication unit configured to receive raw data from a blood pressure measurement apparatus; and a controller configured to determine a level of rareness of the raw data, and to determine whether to transmit the raw data according to the determined level of rareness of the raw data.

The controller may determine the level of rareness of the raw data based on determination criteria including an age of a user using the blood pressure measurement apparatus, the user's medical history, a blood pressure measuring environment, and an aspect of the raw data.

The gateway may further include a display unit configured to receive a command from the controller to display a message for getting confirmation on whether to transmit the raw data.

The communication unit may transmit the raw data to a server if a command for approving transmission of the raw data is received.

In accordance with one or more embodiments, a system may include: a blood pressure measurement apparatus configured to acquire raw data that is used as base information for measurement of blood pressure; and a server configured to receive the raw data, to determine a level of rareness of the raw data, and to determine whether to give a reward for provision of the raw data according to the determined level of rareness of the raw data.

The server may include: a data receiver configured to receive raw data; and a rareness determiner configured to determine a level of rareness of the raw data based on determination criteria including an age of a user using the blood pressure measurement apparatus, the user's medical history, a blood pressure measuring environment, and an aspect of the raw data, and to allocate a weight to the raw data according to the determined level of rareness of the raw data.

The rareness determiner may determine, if the weight allocated to the raw data exceeds a reference value, that a reward for provision of the raw data should be given.

If the raw data is redundancy data about the same user, the rareness determiner may allocate no weight to the raw data.

The system may further include a gateway configured to receive raw data transmitted from the blood pressure measurement apparatus, and to transmit the received raw data to the server.

In accordance with one or more embodiments, a method may include: at a blood pressure measurement apparatus, acquiring raw data for estimating blood pressure from a user; at the blood pressure measurement apparatus, transmitting the raw data to a server; and at the server, determining a level of rareness of the raw data, and determining whether to give a reward for provision of the raw data according to the determined level of rareness of the raw data.

The method may further include, at the blood pressure measurement apparatus, displaying, if acquiring the raw data, a message for getting confirmation on whether to transmit the raw data to the server.

The transmitting of the raw data to the server may include, at the blood pressure measurement apparatus, transmitting the raw data to the server if a command for approving transmission of the raw data to the server is received.

The determining of the level of rareness of the raw data and the determining of whether to give the reward for provision of the raw data according to the determined level of rareness of the raw data may include: at the server, determining the level of rareness of the raw data based on determination criteria including an age of a user using the blood pressure measurement apparatus, the user's medical history, a blood pressure measuring environment, and an aspect of the raw data; at the server, allocating a weight to the raw data according to the determined level of the rareness; and at the server, determining, if the weight allocated to the raw data exceeds a reference value, that a reward for provision of the raw data should be given.

The allocating of the weight to the raw data according to the determined level of the rareness may include allocating no weight to the raw data if the raw data is redundancy data about the same user.

In accordance with one or more embodiments, a method may include: at a blood pressure measurement apparatus, acquiring raw data for estimating blood pressure from a user, and transmitting the raw data to a gateway; at the gateway, transmitting the raw data to a server; and at the server, determining a level of rareness of the raw data, and determining whether to give a reward for provision of the raw data according to the determined level of the raw data.

The method may include, at the gateway, displaying a message for getting confirmation on whether to transmit the raw data to the server if the raw data is transmitted to the gateway.

The transmitting of the raw data to the server may include, at the gateway, transmitting the raw data to the server if a command for approving transmission of the raw data to the server is received.

The determining of the level of rareness of the raw data and the determining of whether to give the reward for provision of the raw data according to the determined level of rareness of the raw data, may include: determining the level of rareness of the raw data based on determination criteria including an age of a user using the blood pressure measurement apparatus, the user's medical history, a blood pressure measuring environment, and an aspect of the raw data; allocating a weight to the raw data according to the determined level of rareness of the raw data; and determining, if the weight allocated to the raw data exceeds a reference value, that a reward for provision of the raw data should be given.

The allocating of the weight to the raw data according to the determined level of the rareness may include allocating no weight to the raw data if the received raw data is redundancy data about the same user.

According to an aspect of the present invention, it may be possible to acquire raw data required for developing a blood pressure estimation algorithm.

Also, by giving a reward for data provision, raw data may be actively collected.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1 is a conceptual view showing a system for development of a blood pressure estimation algorithm, according to one or more embodiments;
FIG. 2 is a block diagram showing the configuration of a system for development of the blood pressure estimation algorithm according to one or more embodiments, such as the system shown in FIG. 1;
FIG. 3 is a conceptual view showing a system for development of a blood pressure estimation algorithm, according to one or more embodiments;
FIG. 4 is a block diagram showing the configuration of a system for development of the blood pressure estimation algorithm according to one or more embodiments, such as the system shown in FIG. 3; and
FIGS. 5 through 8 are flowcharts showing raw data collecting methods for developing a blood pressure estimation algorithm, according to one or more embodiments.

### DETAILED DESCRIPTION

Reference will now be made in detail to one or more embodiments, illustrated in the accompanying drawings, wherein like reference numerals refer to like elements throughout. In this regard, embodiments of the present invention may be embodied in many different forms and should not be construed as being limited to embodiments set forth herein, as various changes, modifications, and equivalents of the systems, apparatuses and/or methods described herein will be understood to be included in the invention by those of ordinary skill in the art after embodiments discussed herein are understood. Accordingly, embodiments are merely described below, by referring to the figures, to explain aspects of the present invention.

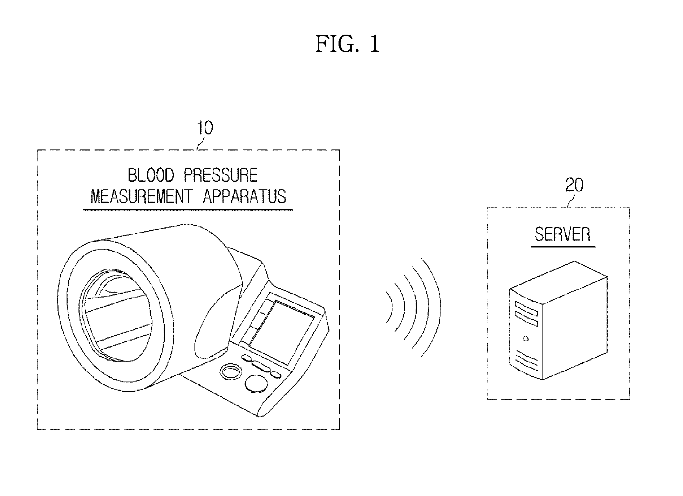

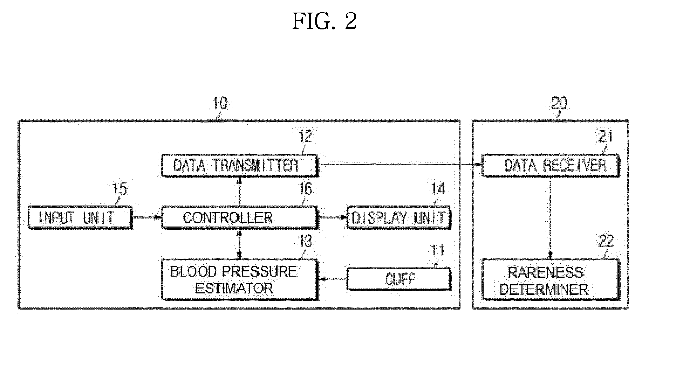

FIG. 1 is a conceptual view showing a system for development of a blood pressure estimation algorithm, according to one or more embodiments, and FIG. 2 is a block diagram showing the configuration of a system for development of the blood pressure estimation algorithm according to one or more embodiments, such as the system shown in FIG. 1.

The system for development of the blood pressure estimation algorithm, according to the embodiment of the present invention, may include a blood pressure measurement apparatus 10 for measuring blood pressure, and a server 20 for receiving raw data transmitted from the blood pressure measurement apparatus 10.

The blood pressure measurement apparatus 10 may a non-invasive blood pressure measurement apparatus, and may include a fully automatic blood pressure measurement apparatus such as may be used at home or hospitals.

The blood pressure measurement apparatus 10 may include an input unit 15 for allowing a user to input a command for operating the blood pressure measurement apparatus 10, a cuff 11 for applying pressure to the user's body region (generally, the user's arm) by being worn around the user's arm or by having the user's arm inserted thereinto, a blood pressure estimator 13 for acquiring raw data by adjusting pressure that is applied through the cuff 11, and estimating the user's blood pressure using a blood pressure estimation algorithm based on the acquired raw data, a display unit 14 for displaying the user's blood pressure estimated by the blood pressure estimator 13, a data transmitter 12 for transmitting the raw data acquired by the blood pressure estimator 13 to the server 20, and a controller 16 for controlling the entire operation of the blood pressure measurement apparatus 10 and determining a level of rareness of the raw data.

The raw data may be a waveform created by the flow of blood between systole and diastole, which is acquired when pressure applied through the cuff 11 is reduced.

The blood pressure estimator 13 may apply the blood pressure estimation algorithm to the raw data acquired by the adjustment of pressure by the cuff 11 to thus calculate systolic pressure, diastolic pressure, and pulse

The systolic pressure, diastolic pressure, and pulse may be displayed on the display unit 14 of the blood pressure measurement apparatus 10, so that the user may check his or her blood pressure through information displayed on the display unit 14.

The data transmitter 12 may transmit the raw data to which the blood pressure estimation algorithm is to be applied, instead of blood pressure information obtained by applying the blood pressure algorithm, to the server 20.

Since the raw data is personal information, the blood pressure measurement apparatus 10 may display a message for getting confirmation on whether to transmit the raw data to the server 20, on the display unit 14, in order to obtain the user's approval before transmitting the raw data.

If a command for approving transmission of the raw data to the server 20 may be received from the user through the input unit 15, the data transmitter 12 may transmit the raw data to the server 20.

Here, the input unit 15 may be configured with a plurality of buttons that perform predetermined functions, and the display unit 14 may be, for example, a general display including a Liquid Crystal Display (LCD), a touch panel, etc. If the display unit 14 is a touch panel, a command for approving transmission of the raw data to the server 20 may be input through the display unit 14.

The server 20 may be a server that may be managed by the manufacturing company of the blood pressure measurement apparatus 10, a server that is managed by public medical institution, or a hospital server. However, in the following description the server 20 is assumed to be a server that is managed by the manufacturing company of the blood pressure measurement apparatus 10.

The server 20 may include a data receiver 21 for receiving the raw data transmitted from the blood pressure measurement apparatus 10, and a rareness determiner 22 for classifying the raw data received by the data receiver 21 according to rareness or importance of the raw data, storing the classified data to construct database, and determining whether to give a reward for data provision according to a level of rareness of the raw data.

Communication between the data transmitter 12 of the blood pressure measurement apparatus 10 and the data receiver 21 of the server 20 amy be carried out through, for example, code division multiple access (CDMA), a wired/wireless LAN, Wibro, 3G, 4G, a public switched telephone network (PSTN), etc. However, the communication is not limited to these.

The rareness determiner 22 may determine a level of rareness of the raw data based on determination criteria including the age of the user using the blood pressure measurement apparatus 10, the user's medical history, a blood pressure measurement environment, the aspect of the raw data, etc.

In order to continue to improve and develop the blood pressure estimation algorithm, it is important to include various and rare raw data in database. That is, in order to develop an algorithm capable of measuring various blood pressures of individuals, rare raw data deviated from sample data is needed in addition to normal raw data.

Accordingly, the rareness determiner 22 may determine a group to which raw data belongs based on determination criteria, such as a user's age, a user's medical history, etc., to determine whether the raw data is rare information deviated from a normal sampling group.

Also, the rareness determiner 22 may determine whether an aspect of the raw data, that is, the waveform type, etc. of the raw data, shows rare information deviated from normal waveforms.

In addition, in order to possibly prevent an error of wrongly determining certain raw data as rare data due to an abnormal blood pressure measurement environment, a blood pressure measurement environment may be included in rareness determination criteria according to which a level of rareness of raw data is determined. For example, in order to possibly prevent an error of wrongly determining, as rare data, raw data acquired when a user's condition is abnormal, such as when blood pressure is measured just after meal or when blood pressure is measured just after exercise, a blood pressure measurement environment may be recognized to be used for determination on rareness of raw data. The blood pressure measurement environment may include various factors, for example, a time at which blood pressure has been measured, a user's temperature, etc. However, the blood pressure measurement environment is not limited to these.

As such, a level of rareness of raw data may be determined, and a weight may be allocated to the raw data according to the determined level of rareness, thereby classifying the raw data.

That is, the raw data may be classified according to its value in such a manner to allocate a greater weight to raw data further deviated from sample data, according to determination based on the rareness determination criteria as described above, and classify raw data according to weights.

Also, the rareness determiner 22 may determine whether the received raw data is redundancy data about the same user. Since already collected data about the same user may not be considered as raw data for improvement and development of an algorithm, such redundancy data may be excluded from database.

Then, the rareness determiner 12 may store the classified raw data to construct database for developing a blood pressure estimation algorithm.

The manufacturing company of the blood pressure measurement apparatus 10 may give a reward to a provider who has provided rare data among the raw data classified by the rareness determiner 22.

That is, after raw data is classified, the rareness determiner 22 may determine, if there is raw data to which a weight exceeding a reference value has been allocated, that a reward for provision of the raw data should be given.

Here, the reference value may be decided in advance to determine a degree of contribution to improvement and development of a blood pressure estimation algorithm. The reward for data provision may be given by various methods.

That is, the reward for data provision may be given in such a manner to offer discounts when the corresponding user purchases other equipment, to pay compensation to the corresponding user, or the like. However, methods of giving a reward for data provision are not limited to these.

Operation of determining a level of rareness of raw data, which may be performed by the server 20, may also be performed by the blood pressure measurement apparatus 10.

The controller 16 of the blood pressure measurement apparatus 10 may determine a level of rareness of raw data based on determination criteria including a user's age, a user's medical history, a blood pressure measurement environment, the aspect of raw data, etc.

The controller 16 may determine a group to which raw data belongs, based on determination criteria, such as a user's age and a user's medical history, to thus determine whether the raw data is rare information deviated from a normal sampling group.

Also, the controller 16 may determine whether an aspect of raw data, that is, the waveform of raw data, is deviated from a normal waveform to thus determine whether the raw data is rare information.

In addition, in order to possibly prevent an error of wrongly determining certain raw data as rare data due to an abnormal blood pressure measurement environment, a blood pressure measurement environment may be included in rareness determination criteria according to which a level of rareness of raw data is determined. For example, in order to possibly prevent an error of wrongly determining, as rare data, raw data acquired when a user's condition is abnormal, such as when blood pressure is measured just after meal or when blood pressure is measured just after exercise, a blood pressure measurement environment may be recognized to be used for determination on rareness of raw data. The blood pressure measurement environment may include various factors, for example, a time at which blood pressure has been measured, a user's temperature, etc. However, the blood pressure measurement environment is not limited to these.

As such, a level of rareness of raw data may be determined, and a weight may be allocated to the raw data according to the determined level of rareness, thereby classifying the raw data.

That is, the raw data may be classified according to its value in such a manner to allocate a greater weight to raw data further deviated from sample data, according to determination based on the rareness determination criteria as described above, and classify raw data according to weights.

However, operation of allocating a weight to raw data may be omitted, or performed by the rareness determiner 22 of the server 20, as described above. Since the weight allocation operation may be mainly used for determination on whether to give a reward for data provision, the weight allocation operation may be performed by the server 20, instead of the blood pressure measurement apparatus 10.

Meanwhile, if the classified raw data may be transmitted to the server 20, the rareness determiner 22 of the server 20 can determine whether the received raw data is redundancy data about the same user, as described above. Since already collected data about the same user may not be considered as raw data for improvement and development of an algorithm, such redundancy data may be excluded from database.

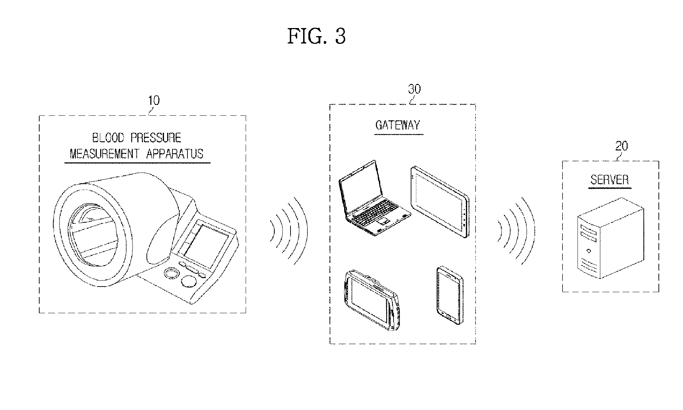

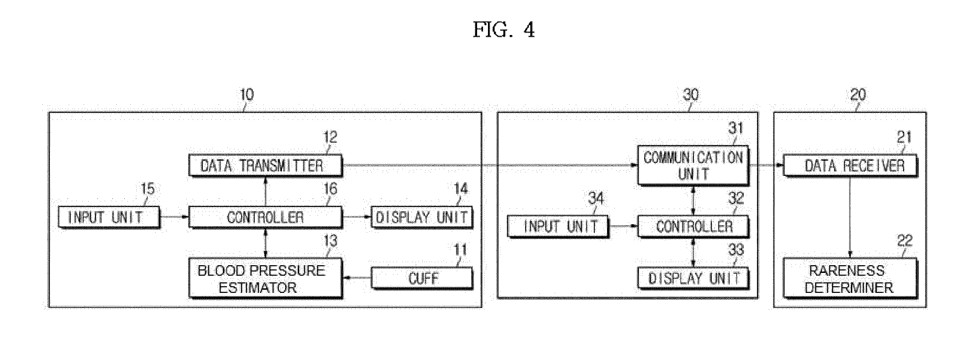

FIG. 3 is a conceptual view showing a system for development of a blood pressure estimation algorithm, according to one or more embodiments, and FIG. 4 is a block diagram showing the configuration of a system for development of the blood pressure estimation algorithm according to one or more embodiments, such as the system shown in FIG. 3.

The system for development of the blood pressure estimation algorithm, according to one or more embodiments, may include a blood pressure measurement apparatus 10 for measuring blood pressure, a gateway 30 for transmitting raw data transmitted from the blood pressure measurement apparatus 10 to a server 20, and the server 20 for receiving the raw data transmitted from the gateway 30.

The blood pressure measurement apparatus 10 may be a non-invasive blood pressure measurement apparatus, and may include a fully automatic blood pressure measurement apparatus such as may be used at home or hospitals.

The blood pressure measurement apparatus 10 may include an input unit 15 for allowing a user to input a command for operating the blood pressure measurement apparatus 10, a cuff 11 for applying pressure to the user's body region (generally, the user's arm) by being worn around the user's arm or by inserting the user's arm thereinto, a blood pressure estimator 13 for acquiring raw data by adjusting pressure that is applied through the cuff 11, and estimating the user's blood pressure using a blood pressure estimation algorithm based on the acquired raw data, a display unit 14 for displaying the user's blood pressure estimated by the blood pressure estimator 13, a data transmitter 12 for transmitting the raw data acquired by the blood pressure estimator 13 to the gateway 30, and a controller 16 for controlling the entire operation of the blood pressure measurement apparatus 10 and determining a level of rareness of the raw data.

The raw data may be a waveform created by the flow of blood between systole and diastole, which is acquired when pressure applied through the cuff 11 is reduced.

The blood pressure estimator 13 may apply the blood pressure estimation algorithm to the raw data acquired by the adjustment of pressure by the cuff 11 to thus calculate systolic pressure, diastolic pressure, and pulse. The systolic pressure, diastolic pressure, and pulse may be displayed on the display unit 14 of the blood pressure measurement apparatus 10, so that the user may check his or her blood pressure through information displayed on the display unit 14.

The data transmitter 12 may transmit the raw data to which the blood pressure estimation algorithm is to be applied, instead of blood pressure information obtained by applying the blood pressure algorithm, to the gateway 30.

When the blood pressure measurement apparatus 10 does not have a function capable of transmitting data to the server 20 located at a long distance, the blood pressure measurement apparatus 10 may transmit raw data to the server 20 via the gateway 30.

In a general healthcare system, since the gateway 30 may perform a function of connecting a biometric information measuring sensor including the blood pressure measurement apparatus 10 to a hospital server, a public medical institute, etc., the blood pressure measurement apparatus 10 may transmit raw data to the server 20 through the function of the gateway 30.

The gateway 30 may include an input unit 34 for receiving a command for operating the gateway 30, a communication unit 31 for receiving raw data from the blood pressure measurement apparatus 10 and transmitting the raw data to the server 20, a controller 32 for controlling the entire operation of the gateway 30, and a display unit 33 for displaying information related to the operation of the gateway 30.

Here, the gateway 30 may be, for example, a type integrated with a display, a set-top box type including an IP TV or cable TV, a smart phone type, a wibro terminal type, a Wifi wireless router type, a PC type including a tablet PC, or a type integrated with medical equipment, etc. However, the gateway 30 is not limited to these.

Communication between the data transmitter 12 of the blood pressure measurement apparatus 10 and the communication unit 31 of the gateway 30 may be carried out through, for example, Bluetooth, infrared data association (IrDA), Wifi, a wired/wireless LAN, Zigbee, Serial, near field communication (NFC), USB communication, etc. However, the communication is not limited to these.

Also, the communication unit 31 of the gateway 30 and the data receiver 21 of the server 20 may be carried out through, for example, CDMA, a wired/wireless LAN, Wibro, 3G, 4G, PSTN, etc. However, the communication is not limited to these.

Meanwhile, since the raw data is personal information, the gateway 30 may display a message for getting confirmation on whether to transmit the raw data to the server 20, on the display unit 33, in order to obtain the user's approval before transmitting the raw data.

If a command for approving transmission of the raw data to the server 20 is received from the user through the input unit 34, the communication unit 31 may transmit the raw data to the server 20.

Here, the input unit 34 may be configured with a plurality of buttons that perform predetermined functions, like the input unit 15 of the blood pressure measurement apparatus 10, and the display unit 33 may be, or eample a general display including a LCD, or a touch panel, etc. If the display unit 33 is a touch panel, a command for approving transmission of the raw data to the server 20 may be input through the display unit 33.

According to one or more embodiments, an operation of obtaining an approval for transmission of raw data to the server 20 may be performed by the blood pressure measurement apparatus 10. That is, operation of obtaining an approval for transmission of raw data to the server 20 may be performed by one of the blood pressure measurement apparatus 10 and the gateway 30.

The server 20 may include a data receiver 21 for receiving the raw data transmitted from the blood pressure measurement apparatus 10, and a rareness determiner 22 for classifying the raw data received by the data receiver 21 according to rareness or importance of the raw data, storing the classified raw data to construct database, and determining whether to give a reward for data provision according to a level of rareness of the raw data.

The rareness determiner 22 may determine a level of rareness of the raw data based on determination criteria including the user's age, the user's medical history, a blood pressure measurement environment, the aspect of the raw data, etc.

In order to continue to improve and develop the blood pressure estimation algorithm, it may be useful to include various and rare raw data in database. That is, in order to develop an algorithm capable of measuring various blood pressures of individuals, rare raw data deviated from sample data may be needed in addition to normal raw data

Accordingly, the rareness determiner 22 may determine a group to which raw data belongs based on determination criteria, such as a user's age, a user's medical history, etc., to determine whether the raw data is rare information deviated from a normal sampling group.

Also, it may be determined whether an aspect of the raw data, that is, the waveform type, etc. of the raw data shows rare information deviated from normal waveforms.

In addition, in order to possibly prevent an error of wrongly determining certain raw data as rare data due to an abnormal blood pressure measurement environment, a blood pressure measurement environment may be included in rareness determination criteria according to which a level of rareness of raw data is determined. For example, in order to prevent an error of wrongly determining, as rare data, raw data acquired when a user's condition is abnormal, such as when blood pressure is measured just after meal or when blood pressure is measured just after exercise, a blood pressure measurement environment may be recognized to be used for determination on rareness of raw data. The blood pressure measurement environment may include various factors, for example, a time at which blood pressure has been measured, a user's temperature, etc. However, the blood pressure measurement environment is not limited to these.

As such, a level of rareness of raw data may be determined, and a weight may be allocated to the raw data according to the determined level of rareness, thereby classifying the raw data.

That is, the raw data may be classified according to its value in such a manner to allocate a greater weight to raw data further deviated from sample data, according to determination based on the rareness determination criteria as described above, and classify raw data according to weights.

Also, the rareness determiner 22 may determine whether the received raw data is redundancy data about the same user. Since already collected data about the same user may not be considered as raw data for improvement and development of an algorithm, such redundancy data may be excluded from database.

The rareness determiner 12 may store the classified raw data to construct database for developing a blood pressure estimation algorithm.

The manufacturing company of the blood pressure measurement apparatus 10 may give a reward to a provider who has provided rare data among the raw data classified by the rareness determiner 22.

That is, after raw data is classified, the rareness determiner 22may determine if a weight allocated to raw data exceeds a reference value, that a reward for provision of the raw data should be given.

Here, the reference value may be decided in advance to determine a degree of contribution to improvement and development of a blood pressure estimation algorithm. The reward for data provision may be given by various methods.

That is, the reward for data provision may be given in such a manner to offer discounts when the corresponding user purchases other equipment, to pay compensation to the corresponding user, or the like. However, methods of giving a reward for data provision are not limited to these.

An operation of determining a level of rareness of raw data, which may be performed by the server 20, may also be performed by the gateway 30.

The controller 32 of the gateway 30 may determine a level of rareness of raw data based on determination criteria including a user's age, a user's medical history, a blood pressure measurement environment, the aspect of raw data, etc.

The controller 32 may determine a group to which raw data belongs, based on determination criteria, such as a user's age and a user's medical history, to thus determine whether the raw data is rare information deviated from a normal sampling group.

Also, the controller 32 may determine whether an aspect of raw data, such as the waveform of raw data is deviated from a normal waveform to thus determine whether the raw data is rare information.

In addition, in order to possibly prevent an error of wrongly determining certain raw data as rare data due to an abnormal blood pressure measurement environment, a blood pressure measurement environment may be included in rareness determination criteria according to which a level of rareness of raw data may be determined. For example, in order to possibly prevent an error of wrongly determining, as rare data, raw data acquired when a user's condition is abnormal, such as when blood pressure is measured just after meal or when blood pressure is measured just after exercise, a blood pressure measurement environment may be recognized to be used for determination on rareness of raw data. The blood pressure measurement environment may include various factors, for example, a time at which blood pressure has been measured, a user's temperature, etc. However, the blood pressure measurement environment is not limited to these.

As such, a level of rareness of raw data may be determined, and a weight may be allocated to the raw data according to the determined level of rareness, thereby classifying the raw data.

That is, the raw data may be classified according to its value in such a manner to allocate a greater weight to raw data further deviated from sample data, according to determination based on the rareness determination criteria as described above, and classify raw data according to weights.

However, an operation of allocating a weight to raw data may be omitted, or performed by the rareness determiner 22 of the server 20, as described above. Since the weight allocation operation may be mainly used for determination on whether to give a reward for data provision, the weight allocation operation may be performed by the server 20, instead of the blood pressure measurement apparatus 10.

Meanwhile, if the classified raw data is transmitted to the server 20, the rareness determiner 22 of the server 20 may determine whether the received raw data is redundancy data about the same user, as described above. Since already collected data about the same user may not be considered as raw data for improvement and development of an algorithm, such redundancy data may be excluded from database.

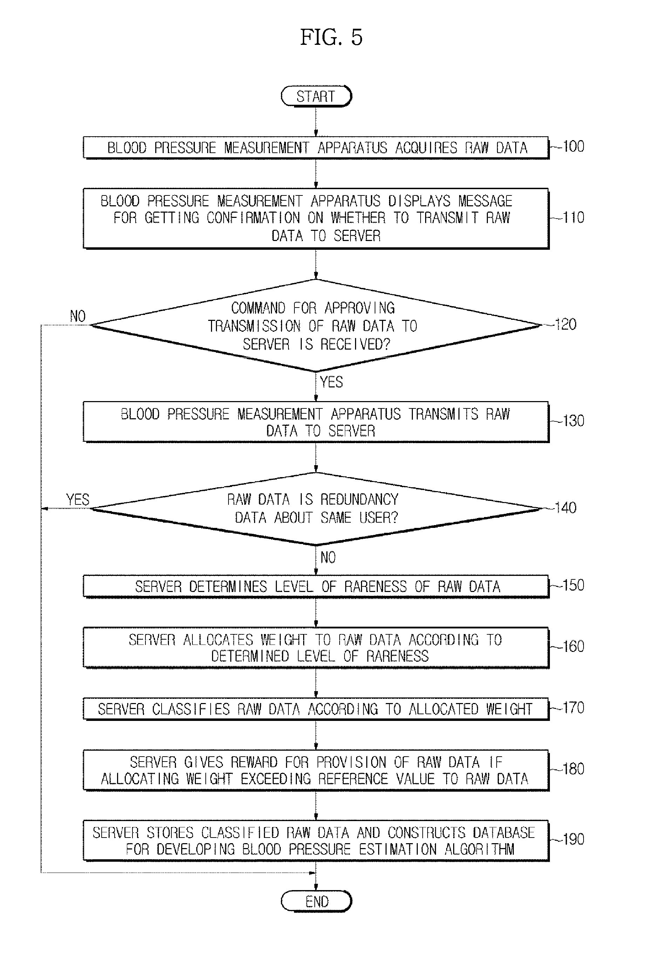

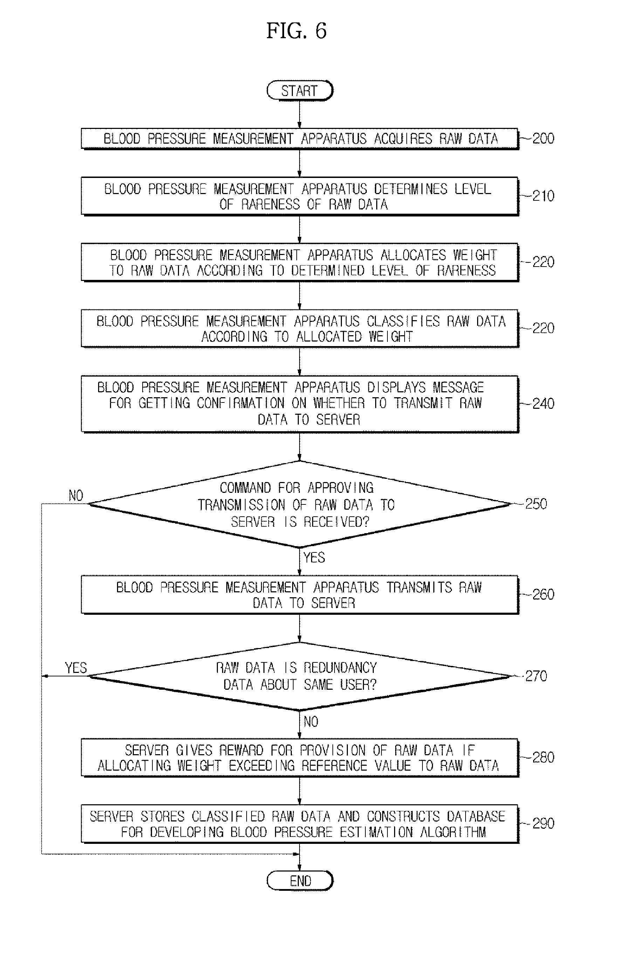

FIGS. 5 and 6 are flowcharts showing raw data collecting methods for developing a blood pressure estimation algorithm, which are performed in a system for development of a blood pressure estimation algorithm according to one or more embodiments, such as the system shown in FIG. 2.

Referring to FIGS. 2 and 5, first, the blood pressure measurement apparatus 10 may acquire raw data (100).

If the blood pressure measurement apparatus 10 acquires the raw data, the blood pressure measurement apparatus 10 may display a message for getting confirmation on whether to transmit the raw data to the server 20, on the display unit 14 (110)

If a command for approving transmission of the raw data to the server 20 is received, the blood pressure measurement apparatus 10 may transmit the raw data to the server 20 (120 and 130).

Since the raw data is personal information, the blood pressure measurement apparatus 10 may display a message for getting confirmation on whether to transmit the raw data to the server 20, on the display unit 14, in order to obtain a user's approval before transmitting the raw data.

If a command for approving transmission of the raw data to the server 20 is received from the user through the input unit 15, the data transmitter 12 may transmit the raw data to the server 20.

Here, the input unit 15 may be configured with a plurality of buttons that perform predetermined functions, and the display unit 14 may be, for example, a general display including a LCD, or a touch panel, etc. If the display unit 14 is a touch panel, a command for approving transmission of the raw data to the server 20 may be input through the display unit 14.

If the server 20 receives the raw data, the server 20 may determine whether the received raw data is redundancy data about the same user (140). Since already collected data about the same user may not be considered as raw data for improvement and development of an algorithm, such redundancy data may be excluded from database.

If the raw data is not redundancy data about the same user, the server 20 may allocate a weight to the raw data according to a level of rareness of the raw data, and may classify the raw data according to the allocated weight (150, 160, and 170).

The server 20 may determine a level of rareness of raw data based on determination criteria including the age of a user using the blood pressure measurement apparatus 10, the user's medical history, a blood pressure measurement environment, the aspect of raw data, etc.

In order to continue to improve and develop the blood pressure estimation algorithm, it may be useful to include various and rare raw data in database. That is, in order to develop an algorithm capable of measuring various blood pressures of individuals, rare raw data deviated from sample data may be needed in addition to normal raw data.

Accordingly, the server 20 may determine a group to which raw data belongs based on determination criteria, such as a user's age, a user's medical history, etc., to determine whether the raw data is rare information deviated from a normal sampling group.

Also, the server 20 may determine whether an aspect of the raw data, such as the waveform type, etc. of the raw data, is rare information deviated from normal waveforms.

In addition, in order to possibly prevent an error of wrongly determining certain raw data as rare data due to an abnormal blood pressure measurement environment, a blood pressure measurement environment may be included in rareness determination criteria according to which a level of rareness of raw data may be determined. For example, in order to possibly prevent an error of wrongly determining, as rare data, raw data acquired when a user's condition is abnormal, such as when blood pressure is measured just after meal or when blood pressure is measured just after exercise, a blood pressure measurement environment may be recognized to be used for determination on rareness of raw data. The blood pressure measurement environment may include various factors, for example, a time at which blood pressure has been measured, a user's temperature, etc. However, the blood pressure measurement environment is not limited to these.

As such, a level of rareness of raw data may be determined, and a weight may be allocated to the raw data according to the determined level of rareness, thereby classifying the raw data.

That is, the raw data may be classified according to its value in such a manner to allocate a greater weight to raw data further deviated from sample data, according to determination based on the rareness determination criteria as described above, and classify raw data according to weights.

That is, after raw data is classified, the server 20 may give a reward for provision of raw data to which a weight exceeding a reference value has been allocated (180).

Here, the reference value may be decided in advance to determine a degree of contribution to improvement and development of a blood pressure estimation algorithm. The reward for data provision may be given by various methods.

That is, the reward for data provision may be given in such a manner to offer discounts when the corresponding user purchases other equipment, to pay compensation to the corresponding user, or the like. However, methods of giving a reward for data provision are not limited to these.

The server 20 may store the classified raw data to construct database for developing a blood pressure estimation algorithm (190).

Referring to FIGS. 2 and 6, the blood pressure measurement apparatus 10 may acquire raw data (200).

If the raw data is acquired, the blood pressure measurement apparatus 10 may determine a level of rareness of the raw data, may allocate a weight to the raw data according to the determined level of rareness of the raw data, and may classify the raw data according to the allocated weight (210, 220, and 230).

The blood pressure measurement apparatus 10 may determine a level of rareness of the raw data based on determination criteria including the age of a user using the blood pressure measurement apparatus 10, the user's medical history, a blood pressure measurement environment, the aspect of the raw data, etc.

In order to continue to improve and develop the blood pressure estimation algorithm, it may be useful to include various and rare raw data in database. That is, in order to develop an algorithm capable of measuring various blood pressures of individuals, rare raw data deviated from sample data may be needed in addition to normal raw data.

Accordingly, the blood pressure measurement apparatus 10 may determine a group to which raw data belongs based on determination criteria, such as the user's age, the user's medical history, etc., to determine whether the raw data is rare information deviated from a normal sampling group.

Also, the blood pressure measurement apparatus 10 may determine whether an aspect of the raw data such as the waveform type, etc. of the raw data, is rare information deviated from normal waveforms.

In addition, in order to possibly prevent an error of wrongly determining certain raw data as rare data due to an abnormal blood pressure measurement environment, a blood pressure measurement environment may be included in rareness determination criteria according to which a level of rareness of raw data is determined. For example, in order to possibly prevent an error of wrongly determining, as rare data, raw data acquired when a user's condition is abnormal, such as when blood pressure is measured just after meal or when blood pressure is measured just after exercise, a blood pressure measurement environment may be recognized to be used for determination on rareness of raw data. The blood pressure measurement environment may include various factors, for example, a time at which blood pressure has been measured, a user's temperature, etc. However, the blood pressure measurement environment is not limited to these.

As such, a level of rareness of raw data may be determined, and a weight may be allocated to the raw data according to the determined level of rareness, thereby classifying the raw data.

That is, the raw data may be classified according to its value in such a manner to allocate a greater weight to raw data further deviated from sample data, according to determination based on the rareness determination criteria as described above, and classify raw data according to weights.

That is, after raw data is classified, the blood pressure measurement apparatus 10 may display a message for getting confirmation on whether to transmit the raw data to the server 20, on the display unit 14 (240).

If a command for approving transmission of the raw data to the server 20 is received from the user through the input unit 15, the blood pressure measurement apparatus 10 may transmit the raw data to the server 20 (250 and 260).

Since the raw data is personal information, the blood pressure measurement apparatus 10 may display a message for getting confirmation on whether to transmit the raw data to the server 20, on the display unit 14, in order to obtain the user's approval before transmitting the raw data.

If a command for approving transmission of the raw data to the server 20 is received from the user through the input unit 15, the data transmitter 12 may transmit the raw data to the server 20.

Here, the input unit 15 may be configured with a plurality of buttons that perform predetermined functions, and the display unit 14 may be, for example, a general display including a LCD, or a touch panel, etc. If the display unit 14 is a touch panel, a command for approving transmission of the raw data to the server 20 may be input through the display unit 14.

If the raw data is received, the server 20 may determine whether the received raw data is redundancy data about the same user. Since already collected data about the same user may not be considered as raw data for improvement and development of an algorithm, such redundancy data may be excluded from database.

If the raw data is not redundancy data about the same user, the server 20 may give a reward for provision of raw data to which a weight exceeding a reference value has been allocated (280).

Here, the reference value may be decided in advance to determine a degree of contribution to improvement and development of a blood pressure estimation algorithm. The reward for data provision may be given by various methods.

That is, the reward for data provision may be given in such a manner to offer discounts when the corresponding user purchases other equipment, to pay compensation to the corresponding user, or the like. However, methods of giving a reward for data provision are not limited to these.

The server 20 may store the classified raw data to construct database for developing a blood pressure estimation algorithm (290).

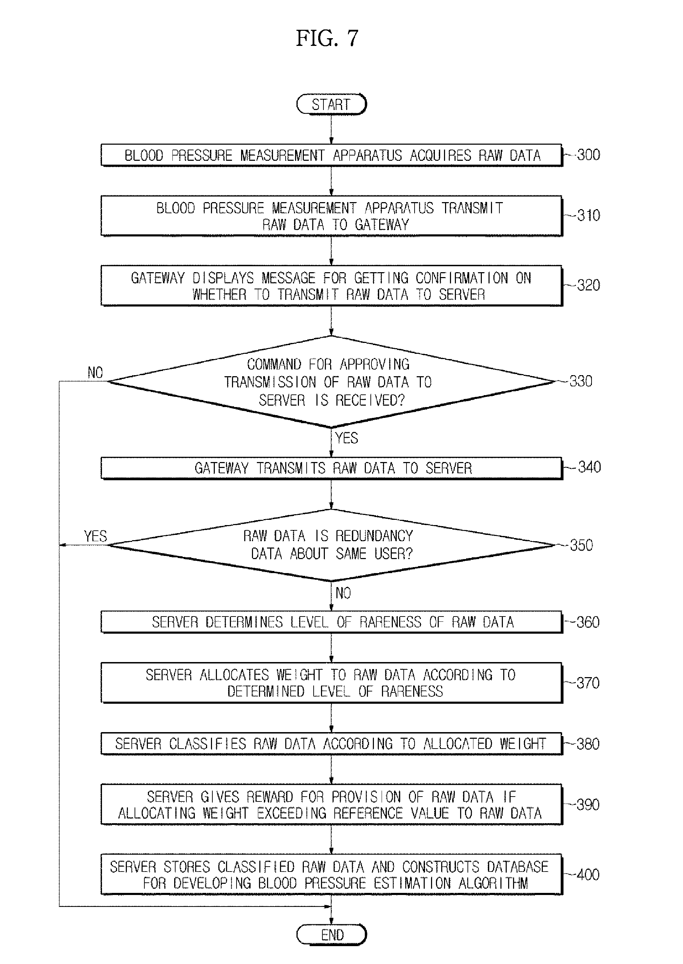

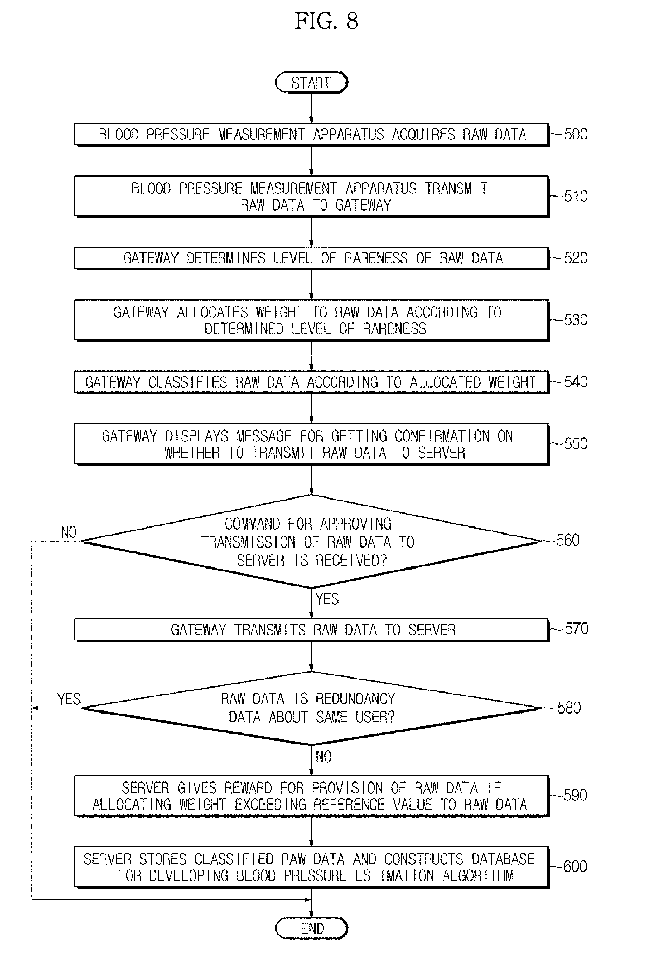

FIGS. 7 and 8 are flowcharts showing raw data collecting methods for developing a blood pressure estimation algorithm according to one or more embodiments, which are performed in system for development of the blood pressure estimation algorithm, such as the system as shown in FIG. 4.

Referring to FIGS. 4 and 7, the blood pressure measurement apparatus 10 may acquire raw data (300).

Then, the blood pressure measurement apparatus 10 may transmit the raw data to the gateway 30 (310).

If the raw data is received, the gateway 30 may display a message for getting confirmation on whether to transmit the raw data to the server 20, on the display unit 33.

If a command for approving transmission of the raw data to the server 20 is received, the gateway 30 may transmit the raw data to the server 20 (330 and 340).

Since the raw data is personal information, the gateway 30 may display a message for getting confirmation on whether to transmit the raw data to the server 20, on the display unit 33, in order to obtain a user's approval before transmitting the raw data.

If a command for approving transmission of the raw data to the server 20 is received from the user through the input unit 34, the communication unit 31 may transmit the raw data to the server 20.

Here, the input unit 34 may be configured with a plurality of buttons that perform predetermined functions, and the display unit 33 may be, for example, a general display including a LCD, or a touch panel, etc. If the display unit 33 is a touch panel, a command for approving transmission of the raw data to the server 20 may be input through the display unit 33.

The following operations 350 through 400 are the same as the operations 140 through 190 of FIG. 5, and accordingly, detailed descriptions thereof will be omitted.

Referring to FIGS. 4 and 8, the blood pressure measurement apparatus 10 may acquire raw data (500).

Then, the blood pressure measurement apparatus 10 may transmit the acquired raw data to the gateway 30 (510).

If the raw data is received, the gateway 30 may determine a level of rareness of the raw data, and may allocate a weight to the raw data according to the determined level of rareness of the raw data, thus classifying the raw data according to the allocated weight (520, 530, and 540).

The gateway 30 may determine a level of rareness of the raw data based on determination criteria including a user's age, a user's medical history, a blood pressure measurement environment, the aspect of the raw data, etc.

In order to continue to improve and develop the blood pressure estimation algorithm, it may be useful to include various and rare raw data in database. That is, in order to develop an algorithm capable of measuring various blood pressures of individuals, rare raw data deviated from sample data may be needed in addition to normal raw data.

Accordingly, the gateway 30 may determine a group to which raw data belongs based on determination criteria, such as a user's age, a user's medical history, etc., to determine whether the raw data is rare information deviated from a normal sampling group.

Also, the gateway 30 may determine whether an aspect of the raw data such as the waveform type, etc. of the raw data, shows rare information deviated from normal waveforms.

In addition, in order to possibly prevent an error of wrongly determining certain raw data as rare data due to an abnormal blood pressure measurement environment, a blood pressure measurement environment may be included in rareness determination criteria according to which a level of rareness of raw data may be determined. For example, in order to possibly prevent an error of wrongly determining, as rare data, raw data acquired when a user's condition is abnormal, such as when blood pressure is measured just after meal or when blood pressure is measured just after exercise, a blood pressure measurement environment may be recognized to be used for determination on rareness of raw data. The blood pressure measurement environment may include various factors, for example, a time at which blood pressure has been measured, a user's temperature, etc. However, the blood pressure measurement environment is not limited to these.

As such, a level of rareness of raw data may be determined, and a weight may be allocated to the raw data according to the determined level of rareness, thereby classifying the raw data.

That is, the raw data may be classified according to its value in such a manner to allocate a greater weight to raw data further deviated from sample data, according to determination based on the rareness determination criteria as described above, and classify raw data according to weights.

If the raw data is classified, the gateway 30 may display a message for getting confirmation on whether to transmit the raw data to the server 20, on the display unit 33 (550).

If a command for approving transmission of the raw data to the server 20 is received, the gateway 30 may transmit the raw data to the server 20 (560 and 570).

Since the raw data is personal information, the gateway 30 may display a message for getting confirmation on whether to transmit the raw data to the server 20, on the display unit 33, in order to obtain the user's approval before transmitting the raw data.

If a command for approving transmission of the raw data to the server 20 is received from the user through the input unit 34, the communication unit 31 may transmit the raw data to the server 20.

Here, the input unit 34 may be configured with a plurality of buttons that perform predetermined functions, and the display unit 33 may be, for example, a general display including a LCD, or a touch panel, etc. If the display unit 33 is a touch panel, a command for approving transmission of the raw data to the server 20 may be input through the display unit 33.

The following operations 580 through 600 are the same as the operations 270 through 290 of FIG. 6, and accordingly, detailed descriptions thereof will be omitted.

As described above, by providing a system and method capable of collecting raw data for improvement or development of a blood pressure estimation algorithm, a blood pressure estimation algorithm capable of more correctly estimating blood pressure may be provided.

In addition, by giving a reward for data provision, raw data may be actively collected.

In one or more embodiments, any apparatus, system, element, or interpretable unit descriptions herein include one or more hardware devices or hardware processing elements. For example, in one or more embodiments, any described apparatus, system, element, retriever, pre or post-processing elements, tracker, detector, encoder, decoder, etc., may further include one or more memories and/or processing elements, and any hardware input/output transmission devices, or represent operating portions/aspects of one or more respective processing elements or devices. Further, the term apparatus should be considered synonymous with elements of a physical system, not limited to a single device or enclosure or all described elements embodied in single respective enclosures in all embodiments, but rather, depending on embodiment, is open to being embodied together or separately in differing enclosures and/or locations through differing hardware elements.

In addition to the above described embodiments, embodiments can also be implemented through computer readable code/instructions in/on a non-transitory medium, e.g., a computer readable medium, to control at least one processing device, such as a processor or computer, to implement any above described embodiment. The medium can correspond to any defined, measurable, and tangible structure permitting the storing and/or transmission of the computer readable code.

The media may also include, e.g., in combination with the computer readable code, data files, data structures, and the like. One or more embodiments of computer-readable media include: magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Computer readable code may include both machine code, such as produced by a compiler, and files containing higher level code that may be executed by the computer using an interpreter, for example. The media may also be any defined, measurable, and tangible distributed network, so that the computer readable code is stored and executed in a distributed fashion. Still further, as only an example, the processing element could include a processor or a computer processor, and processing elements may be distributed and/or included in a single device.

The computer-readable media may also be embodied in at least one application specific integrated circuit (ASIC) or Field Programmable Gate Array (FPGA), as only examples, which execute (e.g., processes like a processor) program instructions.

While aspects of the present invention has been particularly shown and described with reference to differing embodiments thereof, it should be understood that these embodiments should be considered in a descriptive sense only and not for purposes of limitation. Descriptions of features or aspects within each embodiment should typically be considered as available for other similar features or aspects in the remaining embodiments. Suitable results may equally be achieved if the described techniques are performed in a different order and/or if components in a described system, architecture, device, or circuit are combined in a different manner and/or replaced or supplemented by other components or their equivalents.

Thus, although a few embodiments have been shown and described, with additional embodiments being equally available, it would be appreciated by those skilled in the art that changes may be made in these embodiments without departing from the principles and spirit of the invention, the scope of which is defined in the claims and their equivalents.

## Claims

1. A blood pressure measurement apparatus comprising:
a blood pressure estimator configured to acquire raw data for estimating blood pressure, and to estimate blood pressure from the raw data according to a blood pressure estimation algorithm; and
a controller configured to determine a level of rareness of the raw data, and to determine whether to transmit the raw data according to the determined level of rareness of the raw data.

2. The blood pressure measurement apparatus according to claim 1, wherein the controller determines the level of rareness of the raw data based on determination criteria including at least one of an age of a user using the blood pressure measurement apparatus, the user's medical history, a blood pressure measuring environment, and an aspect of the raw data.

3. The blood pressure measurement apparatus according to claim 1 or 2, further comprising a display unit configured to display the estimated blood pressure, and to receive a command from the controller to display a message for getting confirmation on whether to transmit the raw data.

4. The blood pressure measurement apparatus according to claim 1,2 or 3, further comprising a data transmitter configured to transmit the raw data if a command for approving transmission of the raw data is received.

5. The blood pressure measurement apparatus according to claim 4, wherein the data transmitter transmits the raw data to a server or a gateway.

6. A gateway comprising:
a communication unit configured to receive raw data from a blood pressure measurement apparatus; and
a controller configured to determine a level of rareness of the raw data, and to determine whether to transmit the raw data according to the determined level of rareness of the raw data.

7. The gateway according to claim 6, wherein the controller determines the level of rareness of the raw data based on determination criteria including at least one of an age of a user using the blood pressure measurement apparatus, the user's medical history, a blood pressure measuring environment, and an aspect of the raw data.

8. The gateway according to claim 6 or 7, further comprising a display unit configured to receive a command from the controller to display a message for getting confirmation on whether to transmit the raw data.

9. The gateway according to claim 6, 7 or 8, wherein the communication unit transmits the raw data to a server if a command for approving transmission of the raw data is received.

10. A method comprising:
at a blood pressure measurement apparatus, acquiring raw data for estimating blood pressure from a user;
at the blood pressure measurement apparatus, transmitting the raw data to a server; and
at the server, determining a level of rareness of the raw data, and determining whether to give a reward for provision of the raw data according to the determined level of rareness of the raw data.

11. The method according to claim 10, further comprising, at the blood pressure measurement apparatus, displaying, if acquiring the raw data, a message for getting confirmation on whether to transmit the raw data to the server.

12. The method according to claim 10 or 11, wherein the transmitting of the raw data to the server comprises, at the blood pressure measurement apparatus, transmitting the raw data to the server if a command for approving transmission of the raw data to the server is received.

13. The method according to claim 10, 11 or 12 wherein the determining of the level of rareness of the raw data and the determining of whether to give the reward for provision of the raw data according to the determined level of rareness of the raw data comprise:
at the server, determining the level of rareness of the raw data based on determination criteria including at least one of an age of a user using the blood pressure measurement apparatus, the user's medical history, a blood pressure measuring environment, and an aspect of the raw data;
at the server, allocating a weight to the raw data according to the determined level of the rareness; and
at the server, determining the reward to be given if the weight allocated to the raw data exceeds a reference value.

14. The method according to claim 13, wherein the allocating of the weight to the raw data according to the determined level of the rareness comprises allocating no weight to the raw data if the raw data is redundancy data about a same user.
